Europäisches Patentamt

**European Patent Office**

Office européen des brevets

(19)

(11) Numéro de publication: **0 091 368**
**B1**

(12) **FASCICULE DE BREVET EUROPEEN**

(45) Date de publication du fascicule du brevet:
**31.01.90**

(21) Numéro de dépôt: **83400671.0**

(22) Date de dépôt: **31.03.83**

(51) Int. Cl.⁴: **A 61 K 7/32**

(54) **Compositions permettant de combattre les méfaits et les inconvénients de la transpiration.**

(30) Priorité: **01.04.82 FR 8205898**

(43) Date de publication de la demande:
**12.10.83 Bulletin 83/41**

(45) Mention de la délivrance du brevet:
**31.01.90 Bulletin 90/5**

(73) Titulaire: **Olivier, Georges Remy Gaston, 12 bis, route de Nantes, F-85340 Olonne sur Mer (FR)**
Titulaire: **Olivier née Estager, Simone Angèle Marie Henriette, 12 bis, route de Nantes, F-85340 Olonne sur Mer (FR)**

(84) Etats contractants désignés:
**BE CH DE FR GB IT LI**

(72) Inventeur: **Olivier, Georges Rémy Gaston, 12 bis, route de Nantes, F-85340 Olonne sur Mer (FR)**

(74) Mandataire: **Ores, Irène et al, CABINET ORES 6, Avenue de Messine, F-75008 Paris (FR)**

(56) Documents cités:
**FR-A- 1 299 401**
**FR-A- 2 414 331**

**Janistyn I, pp 69 et 126, 1973**
**Remington, p. 778, 1975**

**Le dossier contient des informations techniques présentées postérieurement au dépôt de la demande et ne figurant pas dans le présent fascicule.**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

La présente invention est relative à une composition nouvelle permettant de combattre efficacement les méfaits de la transpiration corporelle et plus particulièrement de la transpiration des pieds et d'agir avec succès et pour une longue durée, sur les inconvénients dus à une transpiration même excessive.

La transpiration est favorisée par les vêtements, gants, chaussures et certains équipements spéciaux qui réduisent les possibilités de dissipation de la chaleur de l'organisme en empêchant les déperditions caloriques nécessaires à celui-ci, ce qui a pour résultat un effort physiologique accru, et par voie de conséquence, une transpiration plus abondante, multipliée par l'effort, l'ambiance, l'état.

L'hypéridrose ainsi favorisée provoque la macération et tous ses inconvénients, certains en apparence anodins telles les odeurs désagréables, la mollesse de la peau qui attendrit l'épiderme et provoque des excoriations, des ampoules occasionnées par le frottement et le poids du corps, l'intertrigo par la pression des orteils serrés les uns contre les autres, l'œil de perdrix, les formations de champignons, les mycoses cutanées, même les hémorragies glandulaires, tous inconvénients qui rendent la marche parfois très douloureuse, perturbent également l'équilibre normal du manteau hydrolitique de la peau. Elle produit aussi l'échauffement et l'intolérance des prothèses et contrecarre très souvent la protection contre le froid.

Cette sueur sécrétée par les glandes sudoripares enflammées (développées normalement après la maturité sexuelle) contient des substances azotées, des matières grasses, de l'eau et du sel. Elle peut même contenir des éléments de l'épiderme. Au moment de la sécrétion, la sueur se présente comme un liquide incolore légèrement opalescent ayant une odeur. Elle est normalement de réaction acide en raison de la présence d'acides gras et de phosphates acides de sodium et de potassium. Le corps par sa température et son humidité favorise le développement de certains microorganismes qui, par leur métabolisme, décomposent les matières organiques se trouvant dans la sueur. Les acides gras et leurs produits de dégradation sont les responsables de l'odeur désagréable de la sueur; ces phénomènes indésirables sont provoqués par des bactéries qui prolifèrent d'autant mieux que la transpiration devient plus abondante en conséquence d'une sécrétion excessive des glandes sudoripares trop sollicitées et enflammées.

Les différents produits d'hygiène corporelle antiperspirants ou désodorisants connus jusqu'à ce jour ne s'attaquaient qu'à une, voire quelques unes des causes, méfaits et inconvénients précités et ne pouvaient agir qu'imparfaitement, fort peu, ou partiellement et presque toujours pour de courtes durées, et pouvaient même être sans action vis-à-vis de certaines macérations ou de certains individus. Ces produits connus dans l'Art antérieur, qui n'ont pas pour préoccupation de s'attaquer aux causes de la transpiration et ne se préoccupent pas non plus de l'état dans lequel se trouve l'épiderme après leur utilisation, sont parfois composés, même en ce qui concerne des produits dits d'hygiène corporelle, de substances toxiques (répertoriées dans les tableaux A, B, C établis par le Ministère de la Santé) soumises à des conditions d'emploi réglementées dans les produits cosmétiques ou les produits d'hygiène corporelle.

Le Brevet français n° 2 414 331 au nom de UNILEVER N.V. a pour objet un produit désordorisant à base d'essences de plantes.

Le Brevet français n° 1 299 401 au nom de COLGATE-PALMOLIVE COMPANY a pour objet une composition antisudorale et désodorisante.

Les compositions décrites dans ces Brevets ne correspondent pas à des compositions permettant de combattre simultanément tous les méfaits de la transpiration (macération et ses inconvénients, échauffement).

La présente invention s'est en conséquence donné pour but de fournir une composition apte à combattre simultanément tous les méfaits et inconvénients de la transpiration ainsi que de présenter une très longue durée d'action.

La présente invention a pour objet une composition permettant de combattre les méfaits et les inconvénients de la transpiration des pieds, ayant une longue durée d'action, comprenant en combinaison:

— au moins un agent absorbant présent dans des proportions comprises entre 10 et 40% et sélectionné dans le groupe qui comprend: kaolin, argile, bentonite, carbonate de chaux, chlorhydrate de zirconium et qui est apte à assécher l'excès d'humidité dans des proportions très supérieures à son poids et peut lui-même perdre cette humidité par évaporation;

— au moins un agent antiseptique présent dans des proportions comprises entre 5 et 35% et sélectionné dans le groupe qui comprend: alun, peroxide de zinc, acétotartrate, naphtol, disulfovinate d'aluminium et présente également des propriétés astringentes, antiputrides, d'assainissement et désodorisantes:

— au moins un agent désodorisant présent dans des proportions comprises entre 2 et 20% et sélectionné dans le groupe qui comprend: magnésie calcinée légère, peroxyde, perborate de magnésium, chlorate de sodium, ester d'acide hydrocarboxylique aliphatique, vitamine $B_3$, cyanocobalamine, acide nicotinique, acide $\beta$ pyridine carbonique et présente également des propriétés absorbantes;

— au moins un agent antisudorifique présent dans des proportions comprises entre 2 et 20% et sélectionné dans le groupe qui comprend: acide tannique, chlorhydrol; l'acide tannique présentant également des propriétés antiseptiques et le chlorhydrol présentant également des propriétés absorbantes;

— au moins un agent nutritif et émollient présent dans des proportions comprises entre 2 et 15% et sélectionné dans le groupe qui comprend: amidon de riz, de maïs, de blé et présente également des propriétés absorbantes;

– au moins un agent de support présent dans des proportions comprises entre 10 et 30% et sélectionné dans le groupe qui comprend: silicate de magnésium, talc et est également absorbant, adoucissant et non fermentescible;

– au moins un agent antibactérien et/ou antimycosique présent dans des proportions comprises entre 0 et 7,50% et sélectionné dans le groupe qui comprend: dichlorhydrate de diamthazole, amphotéricine B, mycostatine, acide benzoïque, aldéhyde formique, acide salicylique, undécylénate de zinc, ricinoléate de zinc, parahydroxybenzoate de méthyle, de propyle et de butyle;

– au moins un agent cicatrisant présent dans des proportions comprises entre 0 et 8% et sélectionné dans le groupe qui comprend: allantoïne, urée, aristol, ledit agent éliminant les callosités et agissant sur la régulation cellulaire, tout en hydratant la peau;

– au moins un agent anti-irritant présent dans des proportions comprises entre 0 et 8% sélectionné dans le groupe qui comprend: acide alphacétoglutarique, oxyde et hydroxyde de zinc, oxyde et peroxyde de titane, ledit agent étant également calmant, désodorisant et protecteur de la peau;

– au moins un agent qui augmente l'adhérence à la peau, présent dans des proportions comprises entre 0 et 10%, ledit agent étant du stéarate de magnésium.

Conformément à l'invention, ladite composition comprend, en outre, au moins un agent conservateur présent dans des proportions comprises entre 0 et 3%, un agent vitaminé présent dans des proportions comprises entre 0 et 10%, un agent parfumant approprié, un agent colorant.

Ladite composition comprend avantageusement:

– un agent conservateur de préparation dermatologique qui est choisi dans le groupe qui comprend: prodhyseptine, ester de l'acide orthohydroxybenzoïque;

– un agent vitaminé qui est choisi dans le groupe qui comprend: vitamine $B_5$, $B_2$, $B_3$, PP, L, E, F, P.

Selon un mode de réalisation préféré de la composition conforme à l'invention, l'agent antibactérien et/ou antimycosique est au moins constitué par l'association de parahydroxybenzoate de méthyle, de propyle et de butyle, qui constituent, en outre, conjointement, un agent conservateur des préparations dermatologiques.

Une formule préférée de la composition conforme à l'invention, présente la formulation qualitative et quantitative suivante:

| | | | |
|---|---|---|---|
| kaolin | de | 10,00 à | 40,00% |
| alun de potassium | de | 5,00 à | 35,00% |
| magnésie calcinée légère | de | 2,00 à | 20,00% |
| acide tannique | de | 2,00 à | 20,00% |
| amidon de riz | de | 2,00 à | 15,00% |
| silicate de magnésie | de | 10,00 à | 30,00% |
| ricinoléate ou undécylénate de zinc | de | 0,00 à | 8,00% |
| para-hydroxybenzoate de méthyle | de | 0,00 à | 2,50% |
| para-hydroxybenzoate de propyle | | 0,00 à | 2,50% |
| para-hydroxybenzoate de butyle | de | 0,00 à | 2,50% |
| allantoïne | de | 0,00 à | 8,00% |
| acide α-cétoglutarique | de | 0,00 à | 8,00% |
| stéarate de magnésium | de | 0,00 à | 10,00% |
| acide folique | de | 0,00 à | 4,00% |
| cyanocobalamine | de | 0,00 à | 4,00% |
| acide linolénique | de | 0,00 à | 4,00% |

Une formule particulièrement préférée de la composition conforme à l'invention, présente la formulation qualitative et quantitative suivante:

| | |
|---|---|
| kaolin | 34,00 % environ |
| alun de potassium | 10,00 % environ |
| magnésie calcinée légère | 8,00 % environ |
| acide tannique | 2,00 % environ |
| chlorhydrol | 6,00 % environ |
| amidon de riz | 5,00 % environ |
| talc | 20,00 % environ |
| acide benzoïque | 2,00 % environ |
| para-hydroxybenzoate de méthyle | 0,070% environ |
| para-hydroxybenzoate de propyle | 0,050% environ |
| para-hydroxybenzoate de butyle | 0,030% environ |
| allantoine | 2,00 % environ |

oxyde de zinc . . . . . . . . . . . . . 5,00 % environ
acide α-cétoglutarique . . . . . . . . . . . 0,850% environ
stéarate de magnésium . . . . . . . . . . 5,00 % environ.

La composition conforme à l'invention est préparée sous forme de poudre, d'onguent, de crème, de bâton, de liquide, d'aérosol, de savon, etc. propre à être appliqué sur la peau pour exercer son action de lutte contre les méfaits et inconvénients de la transpiration. La préparation de la composition conforme à l'invention est réalisée par mélange des constituants, éventuellement associés à des supports ou des véhicules appropriés.

La composition conforme à l'invention permet de traiter une macération à n'importe quelle étape de son évolution et d'obtenir d'excellents résultats, dont les effets persistent pendant des durées très prolongées.

Le traitement est très simple et peu contraignant. Il suffit, en général, dans la grande majorité des cas, de mettre au contact de la peau environ 2,5 grammes de ce produit chaque matin, et de l'y laisser la journée; puis de renouveler la même opération six matins consécutifs et cela seulement deux fois par an à chacun des pieds. Exceptionnellement pour des cas particulièrement rebelles trois fois pourront être nécessaires la première année.

La composition qui fait l'objet de la présente invention peut être utilisée dans tous les cas comme désinfectant, bactéricide, déodorant, cicatrisant, désenflamment, absorbant, adoucissant, régénérant, astringent, assouplissant, hydratant, assainissant, émollient, nutritif, anti irritant, antiseptique, antiputride, antifongique, antimycotique, antisudorifique et hygiénique, en particulier contre les méfaits et inconvénients de la transpiration, en poudre, en liquide, en bloc solide, bâton, crème, onguent, fard, lait, vernis, en vapeur fumigène, savon, shampooing, cosmétique, traitement des étoffes, textiles, papiers, matières plastiques et similaires pour pansements, emplâtres, enduits ou imprégnés de toutes sortes et de toutes préparations. Elle peut servir de remède, soin d'entretien, d'embellissement par contact, par massages, par frictions, par applications de toutes sortes du corps et de la peau.

La composition conforme à l'invention présente une grande efficacité à très long terme en raison, non seulement de l'activité propre de chacun de ses constituents, mais surtout de la synergie d'action entre ses différents constituants qui détermine une augmentation réciproque d'activité desdits constituants et préserve ou améliore l'état du derme, de l'épiderme et des glandes sudoripares.

**Revendications**

1. Composition permettant de combattre les méfaits et les inconvénients de la transpiration des pieds, ayant une longue durée d'action, comprenant en combinaison:
– au moins un agent absorbant présent dans des proportions comprises entre 10 et 40% et sélectionné dans le groupe qui comprend: kaolin, argile, bentonite, carbonate de chaux, chlorhydrate de zirconium et qui est apte à assécher l'excès d'humidité dans des proportions très supérieures à son poids et peut lui-même perdre cette humidité par évaporation;
– au moins un agent antiseptique présent dans des proportions comprises entre 5 et 35% et sélectionné dans le groupe qui comprend: alun, peroxyde de zinc, acétotartrate, naphtol, disulfovinate d'aluminium et présente également des propriétés astringentes, antiputrides, d'assainissement et désodorisantes;
– au moins un agent désodorisant présent dans des proportions comprises entre 2 et 20% et sélectionné dans le groupe qui comprend: magnésie calcinée légère, peroxyde, perborate de magnésium, chlorate de sodium, ester d'acide hydroxycarboxylique aliphatique, vitamine $B_3$, cyanocobalamine, acide nicotinique, acide β pyridine carbonique et présente également des propriétés absorbantes;
– au moins un agent antisudorifique présent dans des proportions comprises entre 2 et 20% et sélectionné dans le groupe qui comprend: acide tannique, chlorhydrol; l'acide tannique présentant également des propriétés antiseptiques et le chlorhydrol présentant également des propriétés absorbantes;
– au moins un agent nutritif et émollient présent dans des proportions comprises entre 2 et 15% et sélectionné dans le groupe qui comprend: amidon de riz, de maïs, de blé et présente également des propriétés absorbantes;
– au moins un agent de support présent dans des proportions comprises entre 10 et 30% et sélectionné dans le groupe qui comprend: silicate de magnésium, talc et est également absorbant, adoucissant et non fermentescible,
– au moins un agent antibactérien et/ou antimycosique présent dans des proportions comprises entre 0 et 7,5% et sélectionné dans le groupe qui comprend: dichlorhydrate de diamthazole, amphotéricine B, mycostatine, acide benzoïque, aldéhyde formique, acide salicylique, undécylénate de zinc, ricinoléate de zinc, parahydroxybenzoate de méthyle, de propyle et de butyle;
– au moins un agent cicatrisant présent dans des proportions comprises entre 0 et 8% et sélectionné dans le groupe qui comprend: allantoïne, urée, aristol, ledit agent éliminant les callosités et agissant sur la régulation cellulaire, tout en hydratant la peau;
– au moins un agent anti-irritant présent dans des proportions comprises entre 0 et 8% et sélectionné dans le groupe qui comprend: acide alphacétoglutarique, oxyde et hydroxyde de zinc, oxyde et peroxyde de titane, ledit agent étant également calmant, désodorisant et protecteur de la peau;
– au moins un agent qui augmente l'adhérence à la peau présent dans des proportions comprises entre 0 et 10%, ledit agent étant du stéarate de magnésium.

2. Composition selon la revendication 1, comprenant, en outre, au moins un agent conservateur présent dans des proportions comprises entre 0 et 3%, un agent vitaminé présent dans des proportions comprises entre 0 et 10%, un agent parfumant approprié, un agent colorant.

3. Composition selon la revendication 2, caractérisée en ce que:

– l'agent conservateur de préparation dermatologique est choisi dans le groupe qui comprend: prodhyseptine, ester de l'acide orthohydroxybenzoïque;

– l'agent vitaminé est choisi dans le groupe qui comprend: vitamine B$_5$, B$_2$, B$_3$, PP, L, E, F, P.

4. Composition selon l'une quelconque des revendications précédentes, caractérisée en ce que l'agent antibactérien et/ou antimycosique est au moins constitué par l'association de parahydroxybenzoate de méthyle, de propyle et de butyle, qui constituent, en outre, conjointement, un agent conservateur des préparations dermatologiques.

5. Composition selon l'une quelconque des revendications 1 à 4, caractérisée en ce qu'elle présente la formulation suivante:

| | | | |
|---|---|---|---|
| kaolin | de | 10,00 à | 40,00% |
| alun de potassium | de | 5,00 à | 35,00% |
| magnésie calcinée légère | de | 2,00 à | 20,00% |
| acide tannique | de | 2,00 à | 20,00% |
| amidon de riz | de | 2,00 à | 15,00% |
| silicate de magnésie | de | 10,00 à | 30,00% |
| ricinoléate ou undécylénate de zinc | de | 0,00 à | 8,00% |
| para-hydroxybenzoate de méthyle | de | 0,00 à | 2,50% |
| para-hydroxybenzoate de propyle | de | 0,00 à | 2,50% |
| para-hydroxybenzoate de butyle | de | 0,00 à | 2,50% |
| allantoïne | de | 0,00 à | 8,00% |
| acide α-cétoglutarique | de | 0,00 à | 8,00% |
| stéarate de magnésium | de | 0,00 à | 10,00% |
| acide folique | de | 0,00 à | 4,00% |
| cyanocobalamine | de | 0,00 à | 4,00% |
| acide linolénique | de | 0,00 à | 4,00% |

6. Composition selon l'une quelconque des revendications 1 à 5, caractérisée en ce qu'elle présente la formulation suivante:

| | |
|---|---|
| kaolin | 34,00 % environ |
| alun de potassium | 10,00 % environ |
| magnésie calcinée légère | 8,00 % environ |
| acide tannique | 2,00 % environ |
| chlorhydrol | 6,00 % environ |
| amidon de riz | 5,00 % environ |
| talc | 20,00 % environ |
| acide benzoïque | 2,00 % environ |
| para-hydroxybenzoate de méthyle | 0,070% environ |
| para-hydroxybenzoate de propyle | 0,050% environ |
| para-hydroxybenzoate de butyle | 0,030% environ |
| allantoine | 2,00 % environ |
| oxyde de zinc | 5,00 % environ |
| acide α-cétoglutarique | 0,850% environ |
| stéarate de magnésium | 5,00 % environ. |

## Claims

1. A long-acting composition for combating the damage and disadvantages due to foot perspiration, comprising a combination of:

– at least one absorbent present in proportions of between 10 and 40% and selected from the group comprising kaolin, clay, bentonite, calcium carbonate and zirconium chlorhydrate, said absorbent being capable of drying the excess moisture in proportions very much greater than its own weight and itself being able to lose this moisture by evaporation;

– at least one antiseptic present in proportions of between 5 and 35% and selected from the group comprising alum, zinc peroxide, acetotartrate, naphthol and aluminium disulphovinate, said antiseptic also having astringent, antiputrefactive, disinfecting and deodorizing properties;

– at least one deodorant present in proportions of between 2 and 20% and selected from the group comprising light calcined magnesia, peroxide, magnesium perborate, sodium chlorate, an aliphatic hydroxycarboxylic acid ester, vitamin B₃, cyanocobalamine, nicotinic acid and β-pyridine-carbonic acid, said deodorant also having absorbent properties;

– at least one antiperspirant present in proportions of between 2 and 20% and selected from the group comprising tannic acid and chlorhydrol, tannic acid also having antiseptic properties and chlorhydrol also having absorbent properties;

– at least one nutrient and emollient present in proportions of between 2 and 15% and selected from the group comprising rice starch, maize starch and wheat starch, said nutrient and emollient also having absorbent properties;

– at least one excipient present in proportions of between 10 and 30% and selected from the group comprising magnesium silicate and talc, said excipient also being absorbent, soothing and non-fermentable;

– at least one antibacterial and/or antimycotic agent present in proportions of between 0 and 7.5% and selected from the group comprising diamthazole dihydrochloride, amphotericin B, mycostatin, benzoic acid, formaldehyde, salicyclic acid, zinc undecylenate, zinc ricinoleate and methyl, propyl and butyl parahydroxybenzoate;

– at least one cicatrizing agent present in proportions of between 0 and 8% and selected from the group comprising allantoin, urea and aristol, said agent eliminating callosity and acting on the cell regulation while at the same time hydrating the skin;

– at least one anti-irritant present in proportions of between 0 and 8% and selected from the group comprising alpha-ketoglutaric acid, zinc oxide and hydroxide and titanium oxide and peroxide, said anti-irritant also soothing, deodorizing and protecting the skin; and

– at least one agent for increasing adhesion to the skin, present in proportions of between 0 and 10%, said agent being magnesium stearate.

2. A composition according to Claim 1 which also comprises at least one preservative present in proportions of between 0 and 3%, a vitamin agent present in proportions of between 0 and 10%, an appropriate fragrance and a colourant.

3. A composition according to Claim 2, characterized in that:

– the preservative for dermatological preparations is selected from the group comprising prodhyseptin and an orthohydroxybenzoic acid ester; and

– the vitamin agent is selected from the group comprising vitamins B₅, B₂, B₃, PP, L, E, F and P.

4. A composition according to any one of the preceding claims, characterized in that the antibacterial and/or antimycotic agent at least consists of a combination of methyl, propyl and butyl parahydroxybenzoate, which together also constitute a preservative for dermatological preparations.

5. A composition according to any one of Claims 1 to 4, characterized in that it has the following formulation:

| | | | |
|---|---|---|---|
| kaolin | from | 10.00 to | 40.00% |
| potassium alum | from | 5.00 to | 35.00% |
| light calcined magnesia | from | 2.00 to | 20.00% |
| tannic acid | from | 2.00 to | 20.00% |
| rice starch | from | 2.00 to | 15.00% |
| magnesium silicate | from | 10.00 to | 30.00% |
| zinc ricinoleate or undecylenate | from | 0.00 to | 8.00% |
| methyl parahydroxybenzoate | from | 0.00 to | 2.50% |
| propyl parahydroxybenzoate | from | 0.00 to | 2.50% |
| butyl parahydroxybenzoate | from | 0.00 to | 2.50% |
| allantoin | from | 0.00 to | 8.00% |
| α-ketoglutaric acid | from | 0.00 to | 8.00% |
| magnesium stearate | from | 0.00 to | 10.00% |
| folic acid | from | 0.00 to | 4.00% |
| cyanocobalamine | from | 0.00 to | 4.00% |
| linolenic acid | from | 0.00 to | 4.00% |

6. A composition according to any one of Claims 1 to 5, characterized in that it has the following formulation:

| | |
|---|---|
| kaolin | about 34.00 % |
| potassium alum | about 10.00 % |
| light calcined magnesia | about 8.00 % |
| tannic acid | about 2.00 % |
| chlorhydrol | about 6.00 % |
| rice starch | about 5.00 % |
| talc | about 20.00 % |

| | |
|---|---|
| benzoic acid . . . . . . . . . . . . . . | about 2.00 % |
| methyl parahydroxybenzoate . . . . . . . | about 0.070% |
| propyl parahydroxybenzoate . . . . . . . | about 0.050% |
| butyl parahydroxybenzoate . . . . . . . | about 0.030% |
| allantoin . . . . . . . . . . . . | about 2.00 % |
| zinc oxide . . . . . . . . . . . . . . | about 5.00 % |
| α-ketoglutaric acid . . . . . . . . . . | about 0.850% |
| magnesium stearate . . . . . . . . . . | about 5.00 % |

**Patentansprüche**

1. Mittel zur Bekämpfung von Schmerzen und Unannehmlichkeiten, hervorgerufen durch Transspiration der Füße, das eine lange Wirkungsdauer aufweist und in Kombination miteinander umfaßt:

– mindestens ein absorbierendes Mittel, das in Mengenanteilen von 10 bis 40% vorhanden und ausgewählt ist aus der Gruppe, die Kaolin, Ton, Bentonit, Kalk(Calcium)-carbonat und Zirkoniumchlorhydrat umfaßt, und das geeignet ist, die überschüssige Feuchtigkeit in Mengenanteilen weit über seinem Gewicht aufzunehmen und selbst diese Feuchtigkeit mittels Verdampfung verlieren kann;

– mindestens ein antiseptisches Mittel, das in Mengenanteilen von 5 bis 35% vorhanden und ausgewählt ist aus der Gruppe, die Alaun, Zinkperoxid, Acetotartrat, Naphthol, Aluminiumdisulfovinat umfaßt, und das ebenfalls adstringierende fäulnishindernde, die Gesundung fördernde und desodorisierende Eigenschaften aufweist;

– mindestens ein desodorierendes Mittel, das in Mengenanteilen von 2 bis 20% vorhanden und aus der Gruppe ausgewählt ist, die leichte gebrannte Magnesia, Peroxid, Magnesiumperborat, Natriumchlorat, Ester von aliphatischer Hydroxycarbonsäure(n), Vitamin B$_3$, Cyanocobalamin, Nikotinsäure und Pyridin-β-carbonsäure umfaßt, und das ebenfalls absorbierende Eigenschaften aufweist;

– mindestens ein schweißhinderndes Mittel, das in Mengenanteilen von 2 bis 20% vorhanden und aus der Gruppe ausgewählt ist, die Tannin und Chlorhydrol umfaßt, wobei das Tannin ebenfalls antiseptische Eigenschaften und das Chlorhydrol ebenfalls absorbierende Eigenschaften aufweist;

– mindestens ein nährendes und weichmachendes Mittel, das in Mengenanteilen von 2 bis 15% vorhanden und aus der Gruppe ausgewählt ist, die Reissstärke, Maisstärke und Weizenstärke umfaßt, und ebenfalls absorbierende Eigenschaften aufweist;

– mindestens einen Träger, der in Mengenanteilen von 10 bis 30% vorhanden und aus der Gruppe ausgewählt ist, die Magnesiumsilicat und Talk umfaßt, und der ebenfalls absorbierend, lindernd und nichtfermentierbar ist;

– mindestens ein antibakterielles und/oder antimykotisches Mittel, das in Mengenanteilen von 0 bis 7,5% vorhanden und aus der Gruppe ausgewählt ist, die Diamthazoldichlorhydrat, Amphotericin B, Mycostatin, Benzoesäure, Formaldehyd, Salicylsäure, Zincundecylenat, Zincricinoleat, Methylparahydroxybenzoat, Propylparahydroxybenzoat und Butylparahydroxybenzoat umfaßt;

– mindestens ein narbenbildendes Mittel, das in Mengenanteilen von 0 bis 8% vorhanden und aus der Gruppe ausgewählt ist, die Allantoin, Harnstoff und Aristol umfaßt, wobei dieses Mittel die Schwielen bzw. Hornhaut entfernt und auf die Zellregulierung einwirkt und gleichzeitig die Haut hydratisiert;

– mindestens ein Mittel gegen Reize aller Art, das in Mengenanteilen von 0 bis 8% vorhanden und aus der Gruppe ausgewählt ist, die alpha-Ketoglutarsäure, Zinkoxid und Zinkhydroxid sowie Titanoxid und Titanperoxid umfaßt, wobei dieses Mittel ebenfalls beruhigend, desodorisierend und hautschützend wirkt;

– mindestens ein Mittel, das die Haftung an die Haut verstärkt und in Mengenanteilen von 0 bis 10% vorhanden ist und Magnesiumstearat ist.

2. Mittel nach Anspruch 1, das zusätzlich mindestens ein konservierendes Mittel in Mengenanteilen von 0 bis 3%, ein vitaminhaltiges Mittel in Mengenanteilen von 0 bis 10% und einen geeigneten Duftstoff sowie einen Farbstoff umfaßt.

3. Mittel nach Anspruch 2, dadurch gekennzeichnet, daß:

– das konservierendes Mittel für dermatologische Präparate ausgewählt ist aus der Gruppe, die Prodhyseptin, Ester der Orthohydroxybenzoesäure umfaßt; und

– das vitaminhaltige Mittel ausgewählt ist aus der Gruppe, die die Vitamine B$_5$, B$_2$, B$_3$, PP, L, E, F und P umfaßt.

4. Mittel nach einem der vorangehenden Ansprüche, dadurch gekennzeichnet, daß das antibakterielle und/oder antimykotische Mittel mindestens besteht aus der Kombination von Methylparahydroxybenzoat, Propylparahydroxybenzoat und Butylparahydroxybenzoat, die darüber hinaus gemeinsam ein Konservierungsmittel für dermatologische Präparate darstellt.

5. Mittel nach einem der Ansprüche 1 bis 4, dadurch gekennzeichnet, daß es folgender Rezeptur entspricht:

| | |
|---|---|
| Kaolin . . . . . . . . . . . . . . . . | 10,00 bis 40,00% |
| Kaliumalaun . . . . . . . . . . . . . | 5,00 bis 35,00% |
| leichte gebrannte Magnesia . . . . . . . . | 2,00 bis 20,00% |
| Tannin . . . . . . . . . . . . . . . | 2,00 bis 20,00% |

| | |
|---|---|
| Reisstärke . . . . . . . . . . . . . . . . . . | 2,00 bis 15,00% |
| Magnesiumsilicat . . . . . . . . . . . . . . . | 10,00 bis 30,00% |
| Zinkricinoleat oder -undecylenat . . . . . . | 0,00 bis 8,00% |
| Methylparahydroxybenzoat . . . . . . . . | 0,00 bis 2,50% |
| Propylparahydroxybenzoat . . . . . . . . . | 0,00 bis 2,50% |
| Butylparahydroxybenzoat . . . . . . . . . | 0,00 bis 2,50% |
| Allantoin . . . . . . . . . . . . . . . . . | 0,00 bis 8,00% |
| α-Ketoglutarsäure . . . . . . . . . . . . | 0,00 bis 8,00% |
| Magnesiumstearat . . . . . . . . . . . . . | 0,00 bis 10,00% |
| Folsäure . . . . . . . . . . . . . . . . . | 0,00 bis 4,00% |
| Cyanocobalamin . . . . . . . . . . . . . | 0,00 bis 4,00% |
| Linolensäure . . . . . . . . . . . . . . . | 0,00 bis 4,00% |

6. Mittel nach einem der Ansprüche 1 bis 5, dadurch gekennzeichnet, daß es folgender Rezeptur entspricht:

| | |
|---|---|
| Kaolin . . . . . . . . . . . . . . . . . . | etwa 34,00 % |
| Kaliumalaun . . . . . . . . . . . . . . . . | etwa 10,00 % |
| leichte gebrannte Magnesia . . . . . . . . | etwa 8,00 % |
| Tannin . . . . . . . . . . . . . . . . . | etwa 2,00 % |
| Chlorhydrol . . . . . . . . . . . . . . . | etwa 6,00 % |
| Reisstärke . . . . . . . . . . . . . . . . | etwa 5,00 % |
| Talk . . . . . . . . . . . . . . . . . . | etwa 20,00 % |
| Benzoesäure . . . . . . . . . . . . . . . | etwa 2,00 % |
| Methylparahydroxybenzoat . . . . . . . . | etwa 0,070% |
| Propylparahydroxybenzoat . . . . . . . . . | etwa 0,050% |
| Butylparahydroxybenzoat . . . . . . . . . | etwa 0,030% |
| Allantoin . . . . . . . . . . . . . . . . . | etwa 2,00 % |
| Zinkoxid . . . . . . . . . . . . . . . . . | etwa 5,00 % |
| α-Ketoglutarsäure . . . . . . . . . . . . | etwa 0,850% |
| Magnesiumstearat . . . . . . . . . . . . | etwa 5,00 % |